# EUROPÄISCHE PATENTSCHRIFT

(11) **EP 2 046 332 B1**
(45) Veröffentlichungstag und Bekanntmachung des Hinweises auf die Patenterteilung: **15.12.2010**
(21) Anmeldenummer: 07786239.9
(22) Anmeldetag: 20.07.2007
(51) Int. Cl.: A61K 31/519, A61P 37/00, A61P 37/06, A61K 9/00, A61P 11/06, A61P 17/06, A61P 19/00, A61P 19/02, A61P 25/28

(54) **KONZENTRIERTE METHOTREXAT-LÖSUNGEN**
CONCENTRATED METHOTREXATE SOLUTIONS
SOLUTIONS DE MÉTHOTREXATE CONCENTRÉES

(30) Priorität: 21.07.2006 DE 102006033837
(43) Veröffentlichungstag der Anmeldung: 15.04.2009
(73) Patentinhaber: medac Gesellschaft für klinische Spezialpräparate mbH, 22880 Wedel (DE)
(72) Erfinder: WILL, Heiner, 22559 Hamburg (DE)
(74) Vertreter: Vossius & Partner
(86) Internationale Anmeldenummer: PCT/EP2007/006491
(87) Internationale Veröffentlichungsnummer: WO 2008/009476

(56) Entgegenhaltungen:
- JANSEN M M P M ET AL: "Methotrexate outside the clinic, intramuscular and subcutaneous administration to patients with rheumatoid arthritis" PHARMACEUTISCH WEEKBLAD 19991119 NL, Bd. 134, Nr. 46, 19. November 1999 (1999-11-19), Seiten 1592-1596, XP008094886 ISSN: 0031-6911
- ROTE LISTE SERVICE GMBH: "Rote Liste 1999" 1999, ECV, EDITIO CANTOR VERLAG , AULENDORF , XP002491051 Seite 86 Abstract 86042
- KURNIK D ET AL: "Bioavailability of oral vs. subcutaneous low-dose methotrexate in patients with Crohn's disease" ALIMENTARY PHARMACOLOGY & THERAPEUTICS, BLACKWELL SCIENTIFIC PUBLICATIONS LTD., CAMBRIDGE, GB, Bd. 18, Nr. 1, 1. Juli 2003 (2003-07-01), Seiten 57-63, XP008094889 ISSN: 0269-2813
- HOEKSTRA MONIQUE ET AL: "Bioavailability of higher dose methotrexate comparing oral and subcutaneous administration in patients with rheumatoid arthritis" JOURNAL OF RHEUMATOLOGY, TORONTO, CA, Bd. 31, Nr. 4, 1. April 2004 (2004-04-01), Seiten 645-648, XP008094880 ISSN: 0315-162X
- ZACKHEIM H S: "SUBCUTANEOUS ADMINISTRATION OF METHOTREXATE" JOURNAL OF THE AMERICAN ACADEMY OF DERMATOLOGY, C.V. MOSBY, ST. LOUIS, MO, US, Bd. 26, Nr. 6, 1. Januar 1992 (1992-01-01), Seite 1008, XP008094895 ISSN: 0190-9622

## Beschreibung

Die vorliegende Erfindung betrifft konzentrierte Methotrexat-Lösungen. Insbesondere betrifft die vorliegende Erfindung die Verwendung von Methotrexat zur Herstellung eines subkutan zu verabreichenden Medikaments zur Behandlung von entzündliche Autoimmunerkrankungen, wobei das Methotrexat in einer Konzentration von etwa 50 mg/ml in einem pharmazeutisch verträglichen Lösungsmittel vorliegt.

Der pharmazeutische Wirkstoff N-{4-[(2,4-Diamino-6-pteridinylmethyl)methylamino]-benzoyl}-L-glutaminsäure (Freiname: Methotrexat, kurz: MTX) ist seit den frühen 50iger Jahren bekannt. Methotrexat ist ein Folsäure-Antagonist. Es bewirkt als Antimetabolit der Nukleuisäuresynthese eine intrazelluläre Hemmung der Dehydrofolat-Reduktase (irreversible Bindung) mit konsekutiver Inhibition der Purinsynthese, hemmt LTB₄-Synthese in Neutrophilen, hemmt IL-1-Synthese, supprimiert zellvermittelte Immunität und hemmt Endothelzellproliferation.

Lange Zeit wurde Methotrexat aufgrund seiner Wirkung als Zytostatikum bevorzugt in der Onkologie eingesetzt. Hier wurde es insbesondere für das Mammakarzinom verwendet, allerdings auch bei Kindern für die Behandlung der Leukämie. Für letztere Indikation ist Methotrexat auch heute noch von entscheidender Bedeutung. Frühzeitig wurde auch die Wirksamkeit von Methotrexat bei der Psoriasis bemerkt Da die Psoriasis auch mit einer rheumatoiden Arthritis einhergenen kann, wurde auch dies als Therapieoption Ende der 50iger Jahren erstmals in Einzelfällen beobachtet.

Die rheumatoide Arthritis wird therapeutisch in der Regel zunächst mit schnell schmerzlindernden und kurzfristig entzündungshemmenden Substanzen behandelt. Hierfür sind nicht-steroidale Antirheumatika (NSAR, z.B. der Wirkstoff Diclofenac) und Kortikoide zu nennen. Jedoch beeinflussen diese den eigentlichen Krankheitsverlauf nicht. Bei den meisten Patienten kommen NSAR und Kortikoide nur so lange zum Einsatz, bis die Entzündungen und Schmerzen deutlich abklingen. Anschließend wird oft deren Dosis reduziert oder das Präparat ganz ausgeschlichen.

Einen krankheitsmodifizierenden Effekt bei der rheumatoiden Arthritis haben nur die Disease Modifiying Anti Rheumatic Drugs (DMARD's). Als Beispiele für diese auch als "Basistherapeutika" bezeichneten Wirkstoffe können neben Methotrexat auch Azathioprin, Sulfasalazin und Antimalariamittel genannt werden. Basistherapeutika greifen direkt in das Krankheitsgeschehen ein und können den Krankheitsprozess bremsen, weshalb ein möglichst frühzeitiger Einsatz anzustreben ist. Da es sich bei der rheumatoiden Arthritis um eine chronische Krankheit handelt, sind die Basistherapeutika meist über entsprechend lange Zeiträume einzunehmen, bei guter Wirksamkeit und Verträglichkeit wird die Therapie oft lebenslang fortgesetzt (kontinuierliche Langzeittherapie), wobei die Wirkstoffdosis an den Krankheitsverlauf adaptiert werden kann.

Im Gegensatz zur Chemotherapie bei Tumorerkrankungen wird Methotrexat als Basistherapeutikum zur Behandlung der rheumatoiden Arthritis um ein Vielfaches, vereinzelt bis zu 1000-fach, geringer dosiert, weshalb man auch bei der antirheumatischen Therapie von der "niedrig-dosierten Methotrexat-Therapie" spricht. In der antirheumatischen Therapie ist in Deutschland ein Dosisbereich von 5,0 bis 30,0mg pro Woche üblich, im europäischen Ausland wird bis zu 40,0mg pro Woche dosiert. Sehr wichtig ist, dass Methotrexat nur einmal pro Woche verabreicht wird.

Die Applikation von Methotrexat kann prinzipiell oral wie auch parenteral erfolgen. Allerdings wurde die mittels Tabletten zunächst für lange Zeit oral erfolgte Therapie durch parenterale Formulierungen abgelöst, da festgestellt wurde, dass Methotrexat aus Tabletten unzuverlässiger resorbiert wird und somit keine ausreichende Genauigkeit bei der dosisabhängigen Therapie gewährleistet ist. Zur parenteralen Verabreichung geeignete Zytostatika werden normalerweise durch Auflösen des Wirkstoffs in einem geeigneten Lösungsmittel hergestellt, indem man für jeden Patienten individuell eine spezifische Wirkstoffmenge verwendet. Die Handhabung von Zytostatika und die Zubereitung Zytostatika-enthaltender Arzneimittel ist allerdings nicht unproblematisch und vom Gesetzgeber mit strengen Auflagen versehen. Beispielsweise dürfen Zytostatika außerhalb einer dafür eigens geschaffenen und geeigneten Abzugsanlage nicht zubereitet werden. Da Rheumatologen und Hausärzte in der Regel nicht über entsprechende Einrichtungen verfügen ist es ihnen nicht gestattet, Methotrexat selbst zuzubereiten, wobei bereits das Aufziehen einer Spritze aus einer Flasche (beispielsweise einer Durchstechflache, welche die Wirkstofflösung enthält) als Zubereitung angesehen wird.

Aus diesem Grund wurden Fertigspritzen entwickelt, um den Arbeitsschritt des Aufziehens zu eliminieren. Erstmals wurden entsprechende Fertigspritzen zur subkutane Applikation für die vorliegende Anmelderin europaweit zugelassen. Mit diesen Fertigspritzen ist die Anwendung durch Arzt, medizinisches Personal oder, bei Selbstapplikation, durch den Patienten selber möglich, ohne dass es eines dazwischengeschalteten Apothekers bedarf, der über ein geeignetes Abzugsystem verfügt.

Aus dem Stand der Technik bekannt sind in Bezug auf die Therapie der rheumatoiden Arthritis Fertigspritzen zur parenteralen Verabreichung enthaltend Methotrexat-Lösungen, in welchen der Wirkstoff in einer Konzentration von bis zu 25mg/ml in einem pharmazeutisch verträglichen Lösungsmittel vorliegt (Handelsnamen: Lantarel® der Firma Wyeth, Metex® der Anmelderin), wobei die Injektionslösung Lantarel® der Konzentration 25 mg/ml (Handelsname: Lantarel® FS 25mg) nicht zur subkutanen Applikation zugelassen ist. Methotrexat hat sich mittlerweile zum "Goldstandard" in der Behandlung der rheumatoiden Arthritis entwickelt

Die subkutane Verabreichung von Methotrexat zur Behandlung der rheumatoiden Arthritis, allerdings ebenfalls mit Konzentrationen von maximal 25 mg/ml, ist weiterhin beschrieben in Jansen et al., Pharmaceutisch Weekblad, Jahrgang 1999, Band 134, Nr. 46, Seiten 1592 - 1596, Kurnik et al., Alimentary Pharmacology & Therapeutics, Jahrgang 2003, Band 18, Nr. 1, Seiten 57 - 63, und Zackheim et al., Journal of the American Academy of Dermatology, Jahrgang 1992, and 26, Nr. 6, Seite 1008.

Wie bereits beschrieben, ist es für eine erfolgreiche Basistherapie mit Methotrexat erforderlich, dass dem Rheumapatienten über einen sehr langen Zeitraum, mitunter ein Leben - lang, wöchentlich die geeignete Dosis Methotrexat verabreicht wird. Für die parenterale Verabreichung spricht die gegenüber dem oralen Applikationsweg beschriebene vorteilhaftere Bioverfügbarkeit. Ferner haben insbesondere Kinder eine gewisse Abneigung gegen die Einnahme von Tabletten. Allerdings hat sich herausgestellt, dass gerade die subkutane Verabreichung mit Schwierigkeiten verbunden ist. Bei Therapien mit den aus dem Stand der Technik bekannten Präparaten stellte sich eine ablehnende Haltung seitens der Patienten heraus. Diese beruht auf der Problematik, im wöchentlichen Zeitabstand die erforderliche relativ große Menge an Wirkstofflösung (z.B. bei entsprechender Wirkstoffdosis bis zu 3ml) unter die Haut zu bringen, was insbesondere Kindern, den wöchentlichen Arztbesuch inbegriffren, nicht leicht zu vermitteln ist.

Es besteht somit der Bedarf für pharmazeutische Formulierungen von Methotrexat, welche dem Patienten, einschließlich Kindern, möglichst einfach und schmerzfrei bei guter Bioverfügbarkeit über einen langen Zeitraum hinweg regelmäßig, insbesondere wöchentlich, verabreicht werden kann und somit zu einer hohen "Patienten-Compliance" führt. Vorteilhafterweise sollte sich der Patient selbst die pharmazeutische Formulierung verabreichen können.

Der vorliegenden Erfindung liegt somit die Aufgabe zugrunde, eine pharmazeutische Zubereitung zur Behandlung von entzündlichen Autoimmunerkrankungen, insbesondere der rheumatoiden Arthritis, bereitzustellen, welche die vorstehend beschriebenen Nachteile der aus dem Stand der Technik bekannten Präparate überwindet.

Die der vorliegenden Erfindung zugrunde liegende Aufgabe wird gelöst durch den Gegenstand der anhängenden Patentansprüche.

In einer ersten Ausführungsform stellt die Erfindung die Verwendung von Methotrexat zur Herstellung eines subkutan zu verabreichenden Medikaments zur Behandlung von entzündlichen Autoimmunerkrankungen, wobei das Methotrexat in einer Konzentration von etwa 50mg/ml in einem pharmazeutisch verträglichen Lösungsmittel vorliegt, bereit.

In einer weiteren Ausführungsform stellt die Erfindung Methotrexat zur Verwendung in der Behandlung von entzündlichen Autoimmunerkrankungen bereit, wobei das Methotrexat subkutan zu verabreichen ist und in einer Konzentration von etwa 50mg/ml in einem pharmazeutisch verträglichen Lösungsmittel vorliegt.

Gemäß der vorliegenden Erfindung werden Medikamente bzw. pharmazeutische Lösunssformulierungen bereitgestellt, welche Methotrexat mit einer Konzentration von etwa 50mg/ml in einem pharmazeutisch verträglichen Lösungsmittel umfassen.

Als das pharmazeutisch verträgliche Lösungsmittel können erfindungsgemäß alle Lösungsmittel in Betracht gezogen werden, die pharmazeutisch verträglich sind und nicht mit dem Wirkstoff sowie eventuell weiteren Inhaltsstoffen des Medikaments bzw. der pharmazeutischen Lösungsformulierung unverträglich sind. Erfindungsgemäß sind besonders geeignete pharmazeutisch verträgliche Lösungsmittel Wasser, insbesondere Wasser für Injektionszwecke, sowie Wasser umfassend Isotonisierungszusätze und Kochsalzlösung, insbesondere isotonische Kochsalzlösung. Am meisten bevorzugt ist Wasser für Injektionszwecke. Als beispielhafte Isotonisierungszusätze können lösliche Salze (Natriumchlorid, Kaliumchlorid), Zucker (Glucose, Lactose), Zuckeralkohole (Mannitol, Sorbitol) sowie Kombinationen aus diesen Hilfsstoffen erfindungsgemäß verwendet werden.

Neben Isotonierungszusätzen kann das erfindungsgemäße Medikament ferner im Fachgebiet der pharmazeutischen Lösungsformulierungen übliche Zusatzstoffe beinhalten. Insbesondere kann das erfindungsgemäße Medikament übliche Hilfsstoffe mit folgender Funktionalität enthalten: Eu-/ Isohydrisierung, (Acetat-, Phosphat-, Citratpuffer), Antioxidantien (Ascorbinsäure, im Fachgebiet übliche Schwefelverbindungen), Läsungsvermittlung (Komplexbildner, Solubilisatoren, Cosolventien: z.B. Cyclodextrine, Polyvidon, Polysorbate, Lecithin, Glykocholat), Viskositätserhöhung, pH-Einstellung (Säuren, Basen, bzw. saure oder basische Salze). In einer besonders bevorzugten Ausführungsform liegt der pH-Wert des erfindungsgemäßen Medikaments zwischen 7,5 und 9.

Die erfindungsgemäßen Medikamente sind auf die Behandlung von entzündlichen Autoimmunerkrankungen gerichtet. Der Begriff "entzündliche Autoimmunerkrankung" umfasst sämtliche entzündlichen Autoimmunerkrankungen, die mit Methotrexat sinnvoll therapiert werden können. Als Beispiele für entzündliche Autoimmunerkrankungen, welche mit den erfindungsgemäßen Medikamenten behandelt werden können, können rheumatoide Arthritis, juvenile Arthritiden, Vaskulitiden, Kollagenosen, Morbus Crohn, Colitis Ulcerosa, Asthma bronchiale, Morbus Alzheimer, Multiple Sklerose, Morbus Bechterew, Gelenkarthrosen oder Psoriasis, sowie Psoriasis Arthritis und insbesondere Psoriasis vulgaris vom Plaque-Typ genannt werden. Besonders bevorzugt eignen sich die erfindungsgemäßen Medikamente zur Behandlung von rheumatoider Arthritis, einschließlich juveniler Arthritiden, wie speziell die oligoarthritische und polyarthritische Form der juvenilen Arthritis.

Die Verabreichung der erfindungsgemäßen Medikamente erfolgt subkutan. Insbesondere erfolgt die Applikation der Medikamente subkutan durch Injection. Weiterhin ist es bevorzugt, dass das Medikament in einer Form vorliegt, die es ermöglicht, dass die subkutane Verabreichung des Medikaments durch den Patienten selbst vorgenommen wird (Selbstapplikation). Eine derartige Therapie der subkutanen Selbstapplikation hat sich beispielsweise bei der Verabreichung von Insulin durch den betroffenen Diabetiker selber bewährt und führt zu einer hohen Therapie-Akzeptanz durch den Patienten ("Patienten-Compliance"). Im Falle der Rheumatherapie kann bei Selbstapplikation zudem auf den in der Regel wöchentlich zu erfolgenden Arztbesuch verzichtet werden.

In einer bevorzugten Ausführungsförm der vorliegenden Erfindung ist das erfindungsgemäße Medikament in einer Injektionsvorrichtung zur Einfachapplikation, insbesondere einer Fertigspritze, enthalten. Unter einer Injektionsvorrichtung zur Einfachapplikation ist erfindungsgemäß eine Vorrichtung zu verstehen, welche neben einem Behältnis, welches die erfindungsgemäße pharmazeutische Lösungsformulierung beinhaltet, eine Injektionsnadel (Kanüle) umfasst, wobei durch diese das Medikament dem Patienten appliziert werden kann. Ferner umfasst eine derartige Injektionsvorrichtung eine mechanische Anordnung (z.B. einen Stempel oder eine flexible Blase), mit deren Hilfe das Medikament durch die Injektionsnadel aus dem Behältnis gedrückt werden kann. Eine derartige Injektionsvorrichtung zur Einfachapplikation ist ferner dadurch gekennzeichnet, dass sie eine gewünschte Einzeldosierung des Wirkstoffs enthält und somit bei der Applikation das Behältnis, welches die erfindungsgemäße pharmazeutische Lösungsformulierung beinhaltet, vollständig zu entleeren ist, um die vorgesehene Dosierung zu verabreichen. Aufgrund dieser Tatsache kann gemäß der vorstehenden Ausführungsform in der Regel auf die Beimischung eines Konservierungsmittels zu der pharmazeutischen Lösungsformulierung von Methotrexat verzichtet werden.

In einer erfindungsgemäßen Injektionsvorrichtung zur Einfachapplikation ist vorzugsweise eine Dosis des Wirkstoffs Methotrexat von 5mg bis 40mg, enthalten. Besonders bevorzugt enthält eine Injektionsvorrichtung zur Einfachapplikation gemäß der Erfindung eine Dosis von 5,0, 7,5, 10,0, 12,5, 15,0, 17,5, 20,0, 22,5, 25,0, 27,5, 30,0, 32,5, 35,0, 37,5 oder 40,0mg. Das zur Bereitstellung einer jeweils gewünschten Dosierung erforderliche Flüssigkeitsvolumen, welches in der Injektionsvorrichtung zur Einfachapplikation enthalten sein muss, hängt von der jeweils gewählten Konzentration der Wirkstofflösung ab und erschließt sich dem Fachmann in einfacher Weise. Demnach müsste zur Bereitstellung einer Wirkstoffdosis von 30,0mg bei einer erfindungsgemäßen konzentration an Methotrexat in dem pharmazeutisch verträglichen Lösungsmittel von 50mg/ml eine Injektionsvorrichtung zur Einfachapplikation ein Flüssigkeitsvolumen von 0,6ml enthalten.

Ein besonders bevorzugtes Beispiel für eine erfindungsgemäße Injektionsvorrichtung zur Einfachapplikation ist eine Fertigspritze. Fertigspritzen sind auf dem pharmazeutischen Fachgebiet gut bekannt, insbesondere auch auf dem Gebiet der Therapie der rheumatoiden Arthritis mit Methotrexat. Auf dem deutschen Mark werden bereits Fertigspritzen enthaltend Methotrexat-Lösungen mit Konzentrationen von 7,5mg/ml, 10,0mg/ml und 25mg/ml vertrieben (Handelsnamen: Lantarel® der Firma Wyeth, Metex® der Anmelderin, wobei das Handelsprodukt Lantarel®) FS 25 mg nicht zur subkutanen Applikation zugelassen ist). Obwohl die Bereitstellung von Methotrexat-Lösungen in Fertigspritze, mitunter zur Seibstapplikation, die Patienten-Compliance positiv beeinflusst, haben die zur subkutanen - Applikation zugelassenen Präparate des Standes der Technik den Nachteil, dass, abhängig von der wöchentlich zu verabreichenden Wirkstoffmenge, verhältnismäßig große Flüssigkeitsmengen unter die Haut des Patienten gebracht werden müssen. Bei einer üblichen wöchentlichen Wirkstoffdosis von 30mg bedeutet dies, dass bei der derzeit höchstkonzentrierten Wirkstofflösung des Standes der Technik zur subkutane Applikation, nämlich 10mg/ml (im Handelsprodukt Metex® 10mg/ml der Anmelderin), ein Volumen von 3ml unter die Haut gespritzt werden muss. Diese hohe Flüssigkeitsmenge ist dem Patienten, insbesondere Kindern, mitunter schwer vermittelbar, was zu einer verminderten Patienten-Compliance führt.

Die erfindungsgemäß bereitgestellten Medikamente enthalten dagegen hochkonzentrierte Lösungen des Wirkstoffs Methotrexat, wodurch sich die zu verabreichende Flüssigkeitsmenge bei einer bestimmten wöchentlichen Wirkstoffdosis verringert. Beispielsweise wäre es demnach bei der erfindungsgemäßen Konzentration von 50mg/ml zur Einhaltung einer wöchentlichen Wirkstoffdosis von 30mg, ausreichend, ein Flüssigkeitsvolumen von lediglich 0,6ml subkutan zu verabreichen. Es kann erwartet werden, dass dies positive Auswirkungen auf die Patienten-Compliance hat.

Fertigspritzen sind auf dem pharmazeutischen Fachgebiet gut bekannt und sind erfindungsgemäß nicht besonders eingeschränkt. Erfindungsgemäße Fertigspritzen umfassen beispielsweise auch Einweg-Injektionssysteme, wie das Uniject®-Injektionssystem. In einer Ausführungsform kann die Fertigspritze bereits mit einer geeigneten Kanüle zur subkutanen Injektion versehen sein, in einer alternativen Ausführungsform ist die Fertigspritze zunächst mit einer Gummikappe oder dgl. versehen, die vor der Applikation durch den Arzt, das medizinische Personal oder, bei subkutane Selbstapplikation, durch den Patienten selber durch eine separat steril verpackte Kanüle ersetzt wird.

Vorzugsweise ist die erfindungsgemäße Fertigspritze so ausgestaltet, dass sie zur subkutanen Applikation der Wirkstofflösung geeignet ist, was insbesondere durch Bereitstellung einer zur Subkutaninjektion geeigneten Kanüle bewerkstelligt werden kann. In einer bevorzugteren Ausführungsform ist die Fertigspritze konstruktiv so ausgestaltet, dass auch Rheumapatienten, die eine eingeschränkte manuelle Feinmotorik aufweisen und daher nicht ausnahmslos in der Lage sind, sich selbst ein Medikament mit herkömmlich ausgestalteten Fertigspritzen zu injizieren, eine Selbstapplikation durchführen können. Hierzu sind vorzugsweise insbesondere Stempel und back stopp derart konstruiert und dimensioniert, so dass dem Rheumapatienten die Handhabung erleichtert ist. Derartig ausgestaltete Fertigspritzen sind auf dem Fachgebiet benannt.

In einer weiteren bevorzugten Ausführungsform der vorliegenden Erfindung ist das erfindungsgemäße Medikament in einem Vorratsgefäß enthalten. Unter einem Vorratsgefäß ist erfindungsgemäß jedes auf dem Fachgebiet übliche Behältnis zu verstehen, in welches/welchem das Medikament bzw. die pharmazeutische Lösungsformulierung der Erfindung fachgerecht, d.h. insbesondere steril, abgefühlt und aufbewahrt werden kann. Als Beispiele für Vorratsgefäß im Sinne der Erfindung können eine Durchstechflasche, ein ViaL ein Beutel, eine Glasampulle oder eine Karpule genannt werden. Gemäß einer Ausführungsform der Erfindung muss zur Applikation des Medikaments an den Patienten zunächst die gewünschte Menge an pharmazeutischer Lösungsformulierung mittels einer Injektionsvorrichtung (beispielsweise eine herkömmliche Einwegspritze) aus dem Vorratsgefäß (beispielsweise eine Durchstechflasche) entnommen werden, während gemäß einer alternativen Ausführungsform der Erfindung die pharmazeutische Lösungsformulierung mittels einer Injektionsvorrichtung (beispielsweise ein Pen-Injektor) direkt aus dem Vorratsgefäß (beispielsweise eine Karpule) appliziert werden kann.

In einer bevorzugten Ausführungsform der Erfindung umfasst das Vorratsgefäß neben dem Wirkstoff Methotrexat, welcher in dem pharmazeutisch verträglichen Lösungsmittel gelöst ist, zumindest noch ein Konservierungsmittel. Das erfindungsgemäß verwendbare Konservierungsmittel ist nicht besonders eingeschränkt und ein Fachmann wird ohne Schwierigkeiten in der Lage sein, aus den bekannten Konservierungsmitteln für pharmazeutische Zwecke ein geeignetes auszuwählen. Als bevorzugte Konservierungsmittel können Cresole, Benzylalkohole, Phenylethylalkohole genannt werden. Das Konservierungsmittel dient insbesondere dazu, die bei einer Teilentnahme des Medikaments (beispielsweise durch eine herkömmliche Einwegspritze oder einen Pen-Injektor) in einem erfindungsgemäßen Vorratsgefäß (beispielsweise eine Durchstechflasche oder eine Karpule) verbleibende pharmazeutische Lösungsformulierung zu konservieren.

Die Gesamtdosierungsmenge an dem Wirkstoff Methotrexat in einem erfindungsgemäßen Vorratsgefäß ist nicht besonders begrenzt und wird insbesondere durch die Abmessungen des Voratsgefäßes und somit vom dem Flüssigkeitsvolumen bestimmt, welches das Vorratsgefäß aufnehmen kann. Bevorzugt enthält ein erfindungsgemäßes Vorratsgefäß eine Gesamtdosierungsmenge von 5 bis 5000 mg Methotrexat.

Als bevorzugtes Beispiel für ein Vorratsgefäß, in welchem das erfindungsgemäße Medikament enthalten ist, kann eine Karpule genannt werden. Karpulen, auch Zylinderampullen genannt, sind auf dem Fachgebiet gut bekannt. Unter einer Karpule versteht der Fachmann einen vorzugsweise zylinderförmigen, sterilen Medikamentenbehälter, welcher vorzugsweise aus Glas oder einem möglichst durchsichtigen, inerten Kunststoff (z.B.: Topas®) gefertigt ist. Auf der einen Seite des Karpulenzylinders befindet sich üblicherweise ein beweglicher Endstopfen, auf der gegenüberliegenden Seite eine durchstechbare Membran aus Gummi oder einem vergleichbaren elastischen Verschlussstoff. Zur Applikation wird das in der Karpule befindliche pharmazeutische Präparat durch Einwirken z.B. eines externen Stempels oder Kolbens auf den beweglichen Endstopfen durch eine Kanüle, welche die beschriebene Gummimembran durchsticht aus der Karpule gepresst

Vorzugsweise wird die Verabreichung, des Medikaments aus der Karpule mittels einer Injektionsvorrichtung vorgenommen. In einer besonders bevorzugten Ausführungsform der Erfindung ist demnach die Karpule zur Verabreichung des Medikaments mittels einer Injektionsvorrichtung geeignet. Derartige Injektionsvorrichtungen sind auf dem Fachgebiet gut bekannt. Als eine solche Injektionsvorrichtung ist vorzugsweise ein sogenannter Pen-Injektor ("Pen") zu nennen, in welchen die Karpule eingesetzt werden kann. Pen-Injektoren sehen in der Regel aus wie größere Füllfederhalter (auf englisch "pen") und sind insbesondere Diabetikern geläufig, die sich mit deren Hilfe bequem die notwendige Dosis Insulin verabreichen können. Nach erfolgter Entleerung der eingesetzten Karpule kann in den Pen-Injektor auf einfache Weise eine neue Karpule eingesetzt werden (vergleichbar dem Austausch einer Tintenpatrone in dem zuvor zum Vergleich genannten Füllfederhalter).

Ein erfindungssemäßer Pen-Injektor ist so ausgestattet, dass er zur subkutanen Applikation der Wirkstofflösung geeignet ist, was insbesondere durch Bereitstellung einer zur Subkutaninjektion geeigneten Kanüle bewerkstelligt werden kann. Ferner ist ein erfindungsgemäßer Pen-Injektor sowie die darin umfasste Karpule vorzugsweise derart ausgestaltet, dass eine Mehrfachapplikation von Einzeldosierungen erfolgen kann. Hierzu ist ein erfindungsgemäßer Pen-Injektor vorzugsweise mit einer konstruktiven Vorrichtung versehen (z.B. einem Regelrad), mit deren Hilfe die Einstellung einer bestimmten Dosierung (d.h. konkret die Auswahl eines bestimmten Applikationsvolumens bei bekannter Wirkstoffkonzentration von Methotrexat in der pharmazeutischen Lösungsformulierung) des zu verabreichenden Methotrexats durch den Arzt, das medizinische Personal oder, bei Selbstapplikation, durch den Patienten selbst vorgenommen werden kann. Somit stellt die Erfindung mit dieser Ausführungsform die Möglichkeit bereit, bei Bedarf auch Zwischendosierungen auszuwählen, für die keine sonstigen Vorratsgefäße oder Injektionsvorrichtungen, insbesondere keine Durchstechflaschen oder Fertigspritzen, kommerziell erhältlich sind. Derartig konstruierte Pen-Injektoren sind auf dem Fachgebiet, insbesondere aus dem Insulinbereich, gut bekannt.

Gemäß einer besonders bevorzugten Ausführungsform der Erfindung ist ein erfindungsgemäßer Pen-Injektor derart ausgestaltet, dass die Einzeldosierungen pro Applikation auf jeweils von 5 bis 40mg Methotrexat eingestellt werden kann. Insbesondere kann ein erfindungsgemäßer Pen-Injektor derart eingestellt werden, dass pro Applikation jeweils eine Einzeldosis von 5,0, 7,5, 10,0, 12,5, 15,0, 17,5, 20,0, 22,5, 25,0, 27,5, 30,0, 32,5, 35,0, 37,5 oder 40,0mg verabreicht werden kann.

Die nachfolgenden Beispiele verdeutlichen die Erfindung.

### Beispiele

### Beispiel 1:

Die nachfolgend veranschaulichte Methotrexat-Lösung (Konzentration: 50 mg/ml) wurde unter Zugrundelegung folgender Rezepturmengen hergestellt.

| | |
|---|---|
| Methotrexat: | 1.500g |
| Natriumchlorid: | 120g |
| Natriumhydroxid: | 300g |
| Wasser für Injektionszwecke: | 28.764g |
| Gesamt: | 30.684g = 30 liter |

Zur Herstellung der Lösung (Beispiel 1) wurden etwa 60% des benötigten Wassers für Injektionszwecke (20-25°C) im Ansatzbehälter vorgelegt. Unter eingeschaltetem Rührwerk wurde die angegebene Menge an Natriumchlorid hinzugegeben und vollständig gelöst. Der Behälter und die Lösung wurde mit Stickstoff geflutet wodurch der Restsauerstoff weitgehend verdrängt wurde. Die angegebene Menge an Methotrexat wurde in der Lösung unter weiter eingeschaltetem Rührwerk suspendiert. Der pH-Wert der Lösung wurde unter Verwendung von 1%iger Natronlauge (Hergestellt aus NaOH und Wasser für Injektionszwecke) auf einen Wert zwischen 8,5 bis 8,9 eingestellt. Die Temperatur der Lösung liegt hierbei zwischen 20 bis 30°C. Es entsteht eine klare Lösung, deren pH-Wert stabil zwischen 8,5 und 8,9 liegt. Durch Zusatz von der Restmenge Wasser für Injektionszwecke wurde auf das endgültige Volumen aufgefüllt.

Durch Sterilfiltration über einen 0,22 µm Sterilfilter wurde die Lösung unter Verwendung von Schutzgas (Stickstoff) in die vorgesehenen sterilen Glasbehälter der Glasart 1 (Karpulen oder Fertigspritzen) unter Reinraumbedingungen (Klasse A) gefüllt.

### Beispiel 2:

Die nachfolgend veranschaulichte Methotrexat-Lösung (Konzentration: 50 mg/ml) wurde unter Zugrundelegung folgender Rezepturmengen hergestellt.

| | |
|---|---|
| Methotrexatdinatrium: | 1.645g |
| Natriumchlorid: | 120g |
| Wasser für Injektionszwecke: | ad 30.684g |
| Gesamt: | 30.684g = 30 liter |

Zur Herstellung der Lösung (Beispiel 2) wurden etwa 60% des benötigten Wassers für Injektionszwecke (20-25°C) im Ansatzbehälter vorgelegt. Unter eingeschaltetem Rührwerk wurde die angegebene Menge an Natriumchlorid hinzugegeben und vollständig gelöst. Der Behälter und die Lösung wurde mit Stickstoff geflutet wodurch der Restsauerstoff weitgehend verdrängt wurde. Die angegebene Menge an Methotrexatdinatriumsalz wurde in der Lösung unter weiter eingeschaltetem Rührwerk gelöst. Die Temperatur der Lösung liegt hierbei zwischen 20 bis 30°C. Die Lösung ist klar und der pH-Wert liegt stabil zwischen 8,5 und 8,9. Durch Zusatz von der Restmenge Wasser für Injektionszwecke wurde auf das endgültige Volumen aufgefüllt.

Durch Sterilfiltration über einen 0,22 µm Sterilfilter wurde die Lösung unter Verwendung von Schutzgas (Stickstoff) in die vorgesehenen sterilen Glasbehälter der Glasart 1 (Karpulen oder Fertigspritzen) unter Reinraumbedingungen (Klasse A) gefüllt.

## Patentansprüche

1. Verwendung von Methotrexat zur Herstellung eines subkutan zu verabreichenden Medikaments zur Behandlung von entzündlichen Autoimmunerkrankungen, wobei das Methotrexat in einer Konzentration von etwa 50 mg/ml in einem pharmazeutisch verträglichen Lösungsmittel vorliegt.

2. Verwendung nach Anspruch 1, wobei das pharmazeutisch verträgliche Lösungsmittel aus Wasser, Wasser für Injektionszwecke, Wasser umfassend Isotonisierungszusätze und Kochsalzlösung, insbesondere isotonische Kochsalzlösung, ausgewählt ist.

3. Verwendung nach einem der vorangehenden Ansprüche, wobei die entzündliche Autoimmunerkrankung ausgewählt ist aus rheumatoide Arthritis, juvenile Arthritiden, Vaskulitiden, Kollagenosen, Morbus Crohn, Colitis Ulcerosa, Astma bronchiale, Morbus Alzheimer, Multiple Sklerose, Morbus Bechterew, Gelenkarthrosen oder Psoriasis.

4. Verwendung nach Anspruch 3, wobei die entzündliche Autoimmunerkrankung rheumatoide Arthritis, insbesondere juvenile rheumatoide Arthritis, ist.

5. Verwendung nach einem der vorstehenden Ansprüche, wobei das Medikament in einer zur Verabreichung durch den Patienten selbst geeigneten Form vorliegt.

6. Verwendung nach einem der vorangehenden Ansprüche, wobei das Medikament in einer Injektionsvorrichtung zur Einfachapplikation enthalten ist.

7. Verwendung nach Anspruch 6, wobei die Injektionsvorrichtung eine Fertigspritze ist.

8. Verwendung nach einem der Ansprüche 1 bis 5, wobei das Medikament in einem Vorratsgefäß enthalten ist.

9. Verwendung nach Anspruch 8, wobei das Medikament ferner ein Konservierungsmittel umfasst.

10. Verwendung nach einem der Ansprüche 8 oder 9, wobei das Vorratsgefäß eine Durchstechflasche, ein Vial, ein Beutel, eine Glasampulle oder eine Karpule ist.

11. Verwendung nach Anspruch 10, wobei das Vorratsgefäß eine Karpule ist und diese zur Verabreichung des Medikaments mittels einer Injektionsvorrichtung, insbesondere eines Pen-Injektors, geeignet ist.

12. Verwendung nach Anspruch 11, wobei die Karpule und der Pen-Injektor derart ausgestaltet sind, dass eine Mehrfachapplikation von Einzeldosierungen erfolgen kann.

13. Verwendung nach Anspruch 7, wobei die Fertigspritze eine Dosierung von 5 bis 40 mg, insbesondere 5,0, 7,5, 10,0, 12,5, 15,0, 17,5, 20,0, 22,5, 25,0, 27,5, 30,0, 32,5, 35,0, 37,5 oder 40,0 mg Methotrexat enthält.

14. Verwendung nach einem der Ansprüche 7 oder 13, wobei die Fertigspritze konstruktiv so ausgestaltet ist, dass einem Patienten mit eingeschränkter Feinmotorik die Selbstapplikation ermöglicht wird.

15. Methotrexat zur Verwendung in der Behandlung von entzündlichen Autoimmunerkrankungen, wobei das Methotrexat subkutan zu verabreichen ist und das Methotrexat in einer Konzentration von etwa 50 mg/ml in einem pharmazeutisch verträglichen Lösungsmittel vorliegt.

16. Methotrexat zur Verwendung nach Anspruch 15, wobei das pharmazeutisch verträgliche Lösungsmittel aus Wasser, Wasser für Injektionszwecke, Wasser umfassend Isotonisierungszusätze und Kochsalzlösung, insbesondere isotonische Kochsalzlösung, ausgewählt ist.

17. Methotrexat zur Verwendung nach einem der Ansprüche 15 oder 16, wobei die entzündliche Autoimmunerkrankung ausgewählt ist aus rheumatoide Arthritis, juvenile Arthritiden, Vaskulitiden, Kollagenosen, Morbus Crohn, Colitis Ulcerosa, Astma bronchiale, Morbus Alzheimer, Multiple Sklerose, Morbus Bechterew, Gelenkarthrosen oder Psoriasis.

18. Methotrexat zur Verwendung nach Anspruch 17, wobei die entzündliche Autoimmunerkrankung rheumatoide Arthritis, insbesondere juvenile rheumatoide Arthritis, ist.

19. Methotrexat zur Verwendung nach einem der Ansprüche 15 bis 18, wobei das Methotrexat in einer zur Verabreichung durch den Patienten selbst geeigneten Form vorliegt.

20. Methotrexat zur Verwendung nach einem der Ansprüche 15 bis 19, wobei das Methotrexat in einer Injektionsvorrichtung zur Einfachapplikation enthalten ist.

21. Methotrexat zur Verwendung nach Anspruch 20, wobei die Injektionsvorrichtung eine Fertigspritze ist.

22. Methotrexat zur Verwendung nach einem der Ansprüche 15 bis 19, wobei das Methotrexat in einem Vorratsgefäß enthalten ist.

23. Methotrexat zur Verwendung nach Anspruch 22, wobei das Methotrexat enthaltende Lösungsmittel ferner ein Konservierungsmittel umfasst.

24. Methotrexat zur Verwendung nach einem der Ansprüche 22 oder 23, wobei das Vorratsgefäß eine Durchstechflasche, ein Vial, ein Beutel, eine Glasampulle oder eine Karpule ist.

25. Methotrexat zur Verwendung nach Anspruch 24, wobei das Vorratsgefäß eine Karpule ist und diese zur Verabreichung des Methotrexats einer Injektionsvorrichtung, insbesondere eines Pen-Injektors, geeignet ist.

26. Methotrexat zur Verwendung nach Anspruch 25, wobei die Karpule und der Pen-Injektor derart ausgestaltet sind, dass eine Mehrfachapplikation von Einzeldosierungen erfolgen kann.

27. Methotrexat zur Verwendung nach Anspruch 21, wobei die Fertigspritze eine Dosierung von 5 bis 40 mg, insbesondere 5,0, 7,5, 10,0, 12,5, 15,0, 17,5, 20,0, 22,5, 25,0, 27,5, 30,0, 32,5, 35,0, 37,5 oder 40,0 mg Methotrexat aufweist.

28. Methotrexat zur Verwendung nach einem der Ansprüche 21 oder 27, wobei die Fertigspritze konstruktiv so ausgestaltet ist, dass einem Patienten mit eingeschränkter Feinmotorik die Selbstapplikation ermöglicht wird.

## Claims

1. Use of methotrexate for the production of a medicament to be administered subcutaneously for the treatment of inflammatory autoimmune diseases, wherein the methotrexate is present in a pharmaceutically acceptable solvent at a concentration of about 50 mg/ml.

2. Use according to claim 1, wherein the pharmaceutically acceptable solvent is selected from water, water for injection purposes, water comprising isotonization additives and sodium chloride solution, in particular isotonic sodium chloride solution.

3. Use according to any of the preceding claims, wherein the inflammatory autoimmune disease is selected from rheumatoid arthritis, juvenile arthritides, vasculitides, collagenoses, Crohn's disease, colitis ulcerosa, bronchial asthma, Alzheimer's disease, multiple sclerosis, Bechterew's disease, joint arthroses, or psoriasis.

4. Use according to claim 3, wherein the inflammatory autoimmune disease is rheumatoid arthritis, in particular juvenile rheumatoid arthritis.

5. Use according to any of the preceding claims, wherein the medicament is present in a form suitable for patient self-administration.

6. Use according to any of the preceding claims, wherein the medicament is contained in an injection device for a single application.

7. Use according to claim 6, wherein the injection device is a ready-made syringe.

8. Use according to any of claims 1 to 5, wherein the medicament is contained in a storage container.

9. Use according to claim 8, wherein the medicament furthermore comprises a preservative.

10. Use according to any of claims 8 or 9, wherein the storage container is an injection bottle, a vial, a bag, a glass ampoule, or a carpule.

11. Use according to claim 10, wherein the storage container is a carpule and wherein said carpule is suitable for administering the medicament by means of an injection device, in particular a pen injector.

12. Use according to claim 11, wherein the carpule and the pen injector are provided such that multiple applications of single dosages can be administered.

13. Use according to claim 7, wherein the ready-made syringe contains a dosage of 5 to 40 mg, in particular 5.0, 7.5, 10.0, 12.5, 15.0, 17.5, 20.0, 22.5, 25.0, 27.5, 30.0, 32.5, 35.0, 37.5 or 40.0 mg, of methotrexate.

14. Use according to any of claims 7 or 13, wherein the ready-made syringe is constructed such that it allows patient self-administration by a patient with limited fine motor skills.

15. Methotrexate for use in the treatment of inflammatory autoimmune diseases, wherein the methotrexate is to be administered subcutaneously and the methotrexate is present in a pharmaceutically acceptable solvent at a concentration of about 50 mg/ml.

16. Methotrexate for use according to claim 15, wherein the pharmaceutically acceptable solvent is selected from water, water for injection purposes, water comprising isotonization additives and sodium chloride solution, in particular isotonic sodium chloride solution.

17. Methotrexate for use according to any of claims 15 or 16, wherein the inflammatory autoimmune disease is selected from rheumatoid arthritis, juvenile arthritides, vasculitides, collagenoses, Crohn's disease, colitis ulcerosa, bronchial asthma, Alzheimer's disease, multiple sclerosis, Bechterew's disease, joint arthroses, or psoriasis.

18. Methotrexate for use according to claim 17, wherein the inflammatory autoimmune disease is rheumatoid arthritis, in particular juvenile rheumatoid arthritis.

19. Methotrexate for use according to any of claims 15 to 18, wherein the methotrexate is present in a form suitable for patient self-administration.

20. Methotrexate for use according to any of claims 15 to 19, wherein the methotrexate is contained in an injection device for a single application.

21. Methotrexate for use according to claim 20, wherein the injection device is a ready-made syringe.

22. Methotrexate for use according to any of claims 15 to 19, wherein the methotrexate is contained in a storage container.

23. Methotrexate for use according to claim 22, wherein the methotrexate-containing solvent furthermore comprises a preservative.

24. Methotrexate for use according to any of claims 22 or 23, wherein the storage container is an injection bottle, a vial, a bag, a glass ampoule, or a carpule.

25. Methotrexate for use according to claim 24, wherein the storage container is a carpule and wherein said carpule is suitable for administering the methotrexate by means of an injection device, in particular a pen injector.

26. Methotrexate for use according to claim 25, wherein the carpule and the pen injector are provided such that multiple applications of single dosages can be administered.

27. Methotrexate for use according to claim 21, wherein the ready-made syringe contains a dosage of 5 to 40 mg, in particular 5.0, 7.5, 10.0, 12.5, 15.0, 17.5, 20.0, 22.5, 25.0, 27.5, 30.0, 32.5, 35.0, 37.5 or 40.0 mg, of methotrexate.

28. Methotrexate for use according to any of claims 21 or 27, wherein the ready-made syringe is constructed such that it allows patient self-administration by a patient with limited fine motor skills.

## Revendications

1. Utilisation de méthotrexate pour la fabrication d'un médicament administrable par voie sous-cutanée pour le traitement de maladies auto-immunes inflammatoires, le méthotrexate étant présent à une concentration d'environ 50 mg/ml dans un solvant pharmaceutiquement acceptable.

2. Utilisation selon la revendication 1, le solvant pharmaceutiquement acceptable étant choisi parmi l'eau, l'eau pour injection, l'eau contenant des additifs d'isotonisation et une solution saline, en particulier une solution saline isotonique.

3. Utilisation selon l'une des revendications précédentes, la maladie auto-immune inflammatoire étant choisie parmi l'arthrite rhumatoïde, les arthrites juvéniles, les vasculites, les collagénoses, la maladie de Crohn, la colite ulcéreuse, l'asthme bronchique, la maladie d'Alzheimer, la sclérose en plaques, la maladie de Bechterew, les arthroses articulaires ou le psoriasis.

4. Utilisation selon la revendication 3, la maladie auto-immune inflammatoire étant l'arthrite rhumatoïde, en particulier l'arthrite rhumatoïde juvénile.

5. Utilisation selon l'une des revendications précédentes, le médicament étant présent sous une forme adaptée à l'auto-administration par le patient.

6. Utilisation selon l'une des revendications précédentes, le médicament étant contenu dans un dispositif d'injection pour une application unique.

7. Utilisation selon la revendication 6, le dispositif d'injection étant une seringue pré-remplie.

8. Utilisation selon l'une des revendications 1 à 5, le médicament étant contenu dans un récipient réservoir.

9. Utilisation selon la revendication 8, le médicament contenant en outre un agent de conservation.

10. Utilisation selon l'une des revendications 8 ou 9, le récipient réservoir étant un flacon à percer, une fiole, une poche, une ampoule de verre ou une carpule.

11. Utilisation selon la revendication 10, le récipient réservoir étant une carpule et celle-ci étant adaptée pour une administration du médicament au moyen d'un dispositif d'injection, en particulier d'un stylo injecteur.

12. Utilisation selon la revendication 11, la carpule et le stylo injecteur étant prévus de manière à permettre une application multiple de doses individuelles.

13. Utilisation selon la revendication 7, la seringue pré-remplie contenant une dose de 5 à 40 mg, en particulier 5,0, 7,5, 10,0, 12,5, 15,0, 17,5, 20,0, 22,5, 25,0, 27,5, 30,0, 32,5, 35,0, 37,5 ou 40,0 mg de méthotrexate.

14. Utilisation selon l'une des revendications 7 ou 13, la seringue pré-remplie étant prévue au niveau de sa construction de manière à permettre l'auto-application par un patient à motricité fine réduite.

15. Méthotrexate pour son utilisation pour le traitement de maladies auto-immunes inflammatoires, le méthotrexate étant à administrer par voie sous-cutanée et le méthotrexate étant présent à une concentration d'environ 50 mg/ml dans un solvant pharmaceutiquement acceptable.

16. Méthotrexate pour son utilisation selon la revendication 15, le solvant pharmaceutiquement acceptable étant choisi parmi l'eau, l'eau pour injection, l'eau contenant des additifs d'isotonisation et une solution saline, en particulier une solution saline isotonique.

17. Méthotrexate pour son utilisation selon l'une des revendications 15 ou 16, la maladie auto-immune inflammatoire étant choisie parmi l'arthrite rhumatoïde, les arthrites juvéniles, les vasculites, les collagénoses, la maladie de Crohn, la colite ulcéreuse, l'asthme bronchique, la maladie d'Alzheimer, la sclérose en plaques, la maladie de Bechterew, les arthroses articulaires ou le psoriasis.

18. Méthotrexate pour son utilisation selon la revendication 17, la maladie auto-immune inflammatoire étant l'arthrite rhumatoïde, en particulier l'arthrite rhumatoïde juvénile.

19. Méthotrexate pour son utilisation selon l'une des revendications 15 à 18, le méthotrexate étant présent sous une forme adaptée à l'auto-administration par le patient.

20. Méthotrexate pour son utilisation selon l'une des revendications 15 à 19, le méthotrexate étant contenu dans un dispositif d'injection pour une application unique.

21. Méthotrexate pour son utilisation selon la revendication 20, le dispositif d'injection étant une seringue pré-remplie.

22. Méthotrexate pour son utilisation selon l'une des revendications 15 à 19, le méthotrexate étant contenu dans un récipient réservoir.

23. Méthotrexate pour son utilisation selon la revendication 22, le solvant contenant le méthotrexate contenant en outre un agent de conservation.

24. Méthotrexate pour son utilisation selon l'une des revendications 22 ou 23, le récipient réservoir étant un flacon à percer, une fiole, une poche, une ampoule de verre ou une carpule.

25. Méthotrexate pour son utilisation selon la revendication 24, le récipient réservoir étant une carpule et celle-ci étant adaptée pour une administration du méthotrexate au moyen d'un dispositif d'injection, en particulier d'un stylo injecteur.

26. Méthotrexate pour son utilisation selon la revendication 25, la carpule et le stylo injecteur étant prévus de manière à permettre une application multiple de doses individuelles.

27. Méthotrexate pour son utilisation selon la revendication 21, la seringue pré-remplie contenant une dose de 5 à 40 mg, en particulier 5,0, 7,5, 10,0, 12,5, 15,0, 17,5, 20,0, 22,5, 25,0, 27,5, 30,0, 32,5, 35,0, 37,5 ou 40,0 mg de méthotrexate.

28. Méthotrexate pour son utilisation selon l'une des revendications 21 ou 27, la seringue pré-remplie étant prévue au niveau de sa construction de manière à permettre l'auto-application par un patient à motricité fine réduite.
